# FASCICULE DE BREVET EUROPEEN

(11) **EP 1 937 759 B1**
(45) Date de publication et mention de la délivrance du brevet: **06.04.2011**
(21) Numéro de dépôt: 06820176.3
(22) Date de dépôt: 10.10.2006
(51) Int. Cl.: C08J 7/12

(54) **PROCÉDÉ DE MODIFICATION DE SURFACES DE POLYMERES, NOTAMMENT D 'HYDROXYLATION DE SURFACES DE POLYMÈRES, ET PRODUITS TELS QU' OBTENUS**
VERFAHREN ZUR MODIFIZIERUNG VON POLYMEROBERFLÄCHEN, WIE DIE HYDROXYLIERUNG VON POLMYMEROBERFLÄCHEN, UND DARAUS ERHALTENE PRODUKTE
METHOD FOR THE MODIFICATION OF POLYMER SURFACES, SUCH AS THE HYDROXYLATION OF POLYMER SURFACES, AND PRODUCTS THUS OBTAINED

(30) Priorité: 11.10.2005 FR 0510371
(43) Date de publication de la demande: 02.07.2008
(73) Titulaire: Alchimer, 91300 Massy (FR)
(72) Inventeur: BUREAU, Christophe, F-91260 Juvisy-sur-Orge (FR); PINSON, Jean, F-94120 Fontenay-sous-Bois (FR)
(74) Mandataire: Grosset-Fournier, Chantal Catherine
(86) Numéro de dépôt international: PCT/FR2006/002270
(87) Numéro de publication internationale: WO 2007/042659

(56) Documents cités:
- EP-A1- 0 300 232
- WO-A-2004/043614
- US-A- 3 925 178
- US-A- 4 740 282

## Description

La présente invention a pour objet un procédé de modification de surfaces de polymères, et notamment un procédé d'hydroxylation de surfaces de polymères, ainsi que les surfaces ainsi modifiées.

L'électrogreffage permet la fonctionnalisation de surfaces conductrices et semiconductrices de l'électricité. Un des avantages considérables de l'électrogreffage est que l'énergie qui permet à la fois la formation des liaisons d'interface et la croissance des films arrive "par la surface" : c'est donc la surface elle-même qui est génératrice de sa propre fonctionnalisation. Cette propriété a; par exemple, pour conséquence que les couches électrogreffées épousent avec grande précision la topologie des surfaces sur lesquelles elles ont été réalisées, et ce même à des échelles nanométriques. A l'échelle macroscopique, elle a également pour conséquence que l'électrogreffage délivre des revêtements sur des pièces ayant une forme de complexité arbitraire avec la même qualité partout : partout où la surface est mouillée par la solution d'électrogreffage, il y aura formation d'un film électrogreffé.

Il est évidemment impossible de réaliser l'électrogreffage sur des surfaces d'isolants, tout au moins sous sa forme habituelle, étant donné que l'activation directe d'un isolant est par nature impossible par voie électrique.

Dans le but de proposer des fonctionnalisations de qualité analogue sur tout type de surfaces, il est nécessaire de mettre au point des procédés de greffage sur isolants, en cherchant - soit dans les précurseurs moléculaires, soit dans les techniques d'activation des surfaces - des spécificités qui permettent de conserver les éléments essentiels constatés pour l'électrogreffage: liaison d'interface (covalente ou non), conformité, homogénéité...

Il est intéressant de fonctionnaliser la surface de polymères pour leur conférer des propriétés spécifiques des propriétés d'hydrophilie, d'hydrophobie, d'adsorption ou de non adsorption de protéines ou d'autres molécules biologiques, de fixation de tous types de matériaux organiques ou inorganiques, de collage, plus généralement toute propriété désirable pour l'application souhaitée et pouvant se ramener à une modification des fonctions offertes par la surface de l'objet considéré. Ceci peut être réalisé directement ou par post-fonctionnalisation, après un traitement initial destiné à rendre la surface plus réactive.

La présente invention a pour but de fournir un procédé de préparation de surfaces modifiées à partir de surfaces de polymères, notamment par l'utilisation de radicaux OH• ou OR•.

La présente invention a également pour but de fournir des surfaces de polymères modifiées, notamment rendues hydrophiles, pouvant par la suite être utilisées dans le cadre de la mise en oeuvre de réactions ultérieures de fonctionnalisations.

La présente invention concerne l'utilisation de radicaux RO•, R représentant un atome d'hydrogène, un groupe alkyle comprenant de 2 à 15 atomes de carbone, un groupe acyle -COR' dans lequel R' représente un groupe alkyle comprenant de 2 à 15 atomes de carbone, ou un groupe aroyle -COAr dans lequel Ar représente un groupe aromatique comprenant de 6 à 15 atomes de carbone, pour l'hydroxylation, l'alcoxylation ou l'oxycarbonylation de surfaces de polymères, lesdits polymères étant différents des polymères choisis parmi : le polyméthacrylate de méthyle (PMMA) et les polymères fluorocarbonés lorsque R représente un atome d'hydrogène, ou de mélanges de polymères, notamment hydrophobes, lesdits polymères étant constitués de motifs monomériques dont au moins 50% parmi ceux-ci sont des motifs aliphatiques, lesdits radicaux RO• étant générés par voie électrochimique ou photochimique.

La présente invention concerne également l'utilisation de radicaux hydroxyles HO•, pour l'hydroxylation de surfaces de polymères hydrophobes, lesdits polymères étant constitués de motifs monomériques dont au moins 50% parmi ceux-ci sont des motifs aliphatiques et étant différents des polymères choisis parmi : le polyméthacrylate de méthyle (PMMA) et les polymères fluorocarbonés.

L'expression "hydroxylation de surfaces" désigne la fixation de groupes hydroxyles (-OH) sur lesdites surfaces.

L'expression "alcoxylation de surfaces" désigne la fixation de groupes alcoxy (-OR) sur lesdites surfaces, R étant un groupe alkyle tel que défini ci-dessus.

L'expression "oxycarbonylation de surfaces" désigne la fixation de groupes oxycarbonyles (-OCOR' ou -OCOAr, R' et Ar étant tels que définis ci-dessus) sur lesdites surfaces.

L'expression "mélanges de polymères" désigne un matériau obtenu en mélangeant au moins deux polymères. Par exemple, le polymère Acrylonitrile-Butadiène-Styrène est obtenu en dispersant une phase élastomérique greffée (butadiène) dans une phase styrénique : copolymère styrène-acrylonitrile.

L'expression "motifs monomériques" désigne les motifs qui sont répétés dans le polymère.

L'expression "motifs aliphatiques" désigne des motifs ne comportant pas de groupe aromatiques, c'est-à-dire de groupe cyclique comportant 4n+2 électrons délocalisés sur l'ensemble du cycle.

Selon un mode de réalisation préféré, les polymères hydrophobes auxquels on applique le procédé d'hydroxylation de l'invention sont des polymères dont les atomes de la chaîne principale : carbone, azote ou oxygène, ne sont liés que par des liaisons simples.

L'expression "chaîne principale" désigne l'enchaînement le plus long qu'il est possible de parcourir sur une chaîne polymérique.

Selon un mode de réalisation préféré, les polymères hydrophobes auxquels on applique le procédé d'hydroxylation de l'invention sont des polymères dont les atomes de la chaîne principale : carbone, azote ou oxygène, ne sont liés que par des liaisons simples, les substituants des chaînes latérales n'étant pas des groupes carboxyliques ou leurs esters ou des atomes de fluor, mais étant de préférence des atomes d'hydrogène, des groupes alkyles comprenant de 1 à 6 atomes de carbone, des groupés aryles comprenant de 6 à 14 atomes de carbone et éventuellement des hétéroatomes choisis parmi : N, O et S, des groupes cyano ou des atomes de chlore.

Selon un mode de réalisation préféré, les polymères hydrophobes auxquels on applique le procédé d'hydroxylation de l'invention ne sont pas des polymères siliconés.

La présente invention concerne également l'utilisation de la réaction de Fenton, pour l'hydroxylation, l'alcoxylation ou l'oxycarbonylation de surfaces de polymères, la réaction d'hydroxylation étant effectuée sur des polymères différents des polymères choisis parmi : le polyméthacrylate de méthyle (PMMA) et les polymères fluorocarbonés, ou de mélanges de polymères, lesdits polymères étant constitués de motifs monomériques dont au moins 50% parmi ceux-ci sont des motifs aliphatiques, la réaction de Fenton étant mise en oeuvre par voie électrochimique ou par voie photochimique.

L'expression "réaction de Fenton" désigne la réaction qui permet de produire des radicaux hydroxyles par réaction de l'eau oxygénée avec du fer (II).

Cette réaction peut être représentée par le schéma réactionnel suivant :

Fe²+ + H₂O₂ + H⁺ → Fₑ³⁺ + H₂O + HO° k = 55 M⁻¹ S⁻¹

Elle est notamment décrite dans les articles suivants : Fenton, H., J., H. J. Chem. Soc. 1894, 65, 899 ; Haber, F.; Weiss, J. Proc.Roy. Soc. A. 1934, 134, 332 ; Barb.; W.G.; Baxendale, J., H.; George, P.; Hargrave, K. R. Nature 1949, 163, 692 ; Walling, C.; Weil, T. Int. J. Chem. Kinet. 1974, 6, 507 ; Gallard, H.; DeLaat, J.; Legube, B. Wat. Res. 1999, 33, 2929.

Cette réaction a été également appliquée, dans le cadre de la présente invention, en remplaçant l'eau oxygénée par un peroxyde ROOR, R étant tel que défini ci-dessus.

L'utilisation de la réaction de Fenton pour l'hydroxylation, l'alcoxylation ou l'oxycarbonylation de surface permet d'obtenir des radicaux hydroxyles, - alcoxy ou oxycarbonyles de façon plus simple notamment que les traitements plasma ou les irradiations gamma.

Il est particulièrement avantageux d'utiliser les réactions de Fenton, d'ElectroFenton et de PhotoFenton, car elles s'appliquent aux polymères indépendamment de leur structure chimique : elles sont donc non spécifiques des structures chimiques des polymères.

Selon un mode de réalisation avantageux, la présente invention concerne l'utilisation telle que définie ci-dessus, caractérisée en ce que la réaction de Fenton est mise en oeuvre par voie électrochimique, c'est-à-dire par la mise en oeuvre de la réaction d'ElectroFenton.

La réaction d'ElectroFenton permet de produire en continu des radicaux hydroxyles en régénérant continuellement le Fe²⁺ à la cathode et en réduisant le dioxygène en eau oxygénée sur la même cathode. Cette variante de la réaction de Fenton permet donc de produire de grandes quantités de radicaux en laissant l'électrolyse branchée pendant un temps suffisant.

La réaction d'ElectroFenton (Tomat, R.; Vecchi, A. J Appl. Electrochem. 1971, 1, 185; Oturan, M. A.; Pinson, J. New J. Chem 1992, 16, 705 ; Fang, X.; Pam, X.; Rahman, A. P. Chem.Eur. J. 1995,1,423 ; Gallard, H.; DeLaat, J. Chemosphere 2001, 42, 405 ; Matsue.T., Fujihira, M.; Osa, T. J Electrochem.Soc. 1981, 128, 2565; Fleszar, B.; Sobkoviak, A. Electrochim. Act. 1983, 28, 1315 ; Tzedakis, T.; Savall, A.;Clifton, M., J. J. Appl. Electrochem. 1989, 19, 911 ; Oturan, M. A.; Oturan, N.; Lahitte, C.; Trévin, S. J. Electranal. Chem. 2001, 507, 96 ; Brillas, E.; Casado, J. Chemosphere 2002, 47, 241) est une variante de la réaction de Fenton et consiste en une réaction catalytique où le Fe²⁺ est régénéré en continu à la cathode en même temps que l'oxygène est réduit pour former de l'eau oxygénée.

Le cycle catalytique est décrit ci-dessous :

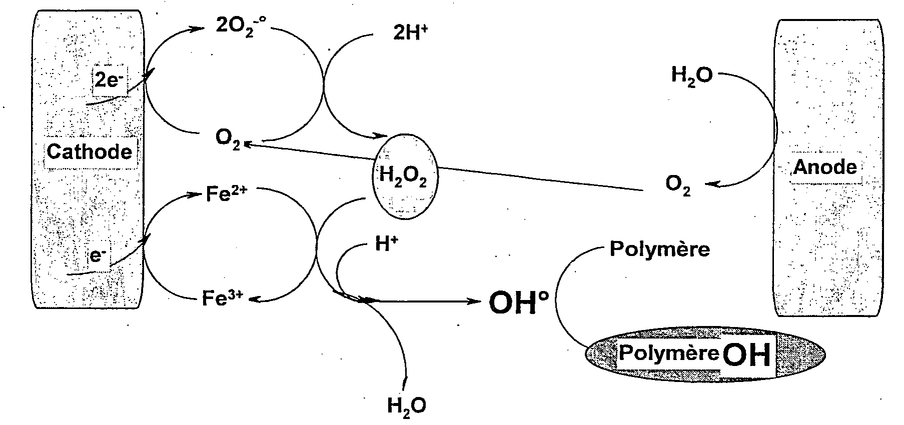

De plus, dans le cadre du procédé de la présente invention, l'oxycarbonylation des surfaces a été effectuée en introduisant directement dans la cellule d'électrolyse le peroxyde et ce sans désoxygénation de la solution.

Selon un autre mode de réalisation avantageux, la présente invention concerne l'utilisation telle que définie ci-dessus, caractérisée en ce que la réaction de Fenton est mise en oeuvre par voie photochimique, c'est-à-dire par la mise en oeuvre de la réaction de PhotoFenton.

La réaction de PhotoFenton (Brillas, E.; Sauleda, R.; Casado, J. J. Electrochem. Soc. 1998, 145, 759) est une variante de la réaction de Fenton, correspondant au mécanisme suivant :

H₂O₂ + Fe^{2+ +} H⁺ → Fe³⁺ + H₂O + HO°

Fe³⁺ + H₂O + hv → Fe²⁺ + HO° + H⁺

H₂O₂ + hv → 20H°

La réaction de PhotoFenton permet de produire des radicaux hydroxyles par irradiation d'une solution aqueuse d'eau oxygénée et de Fe(II). Cette réaction régénère en permanence le Fe(II) et permet donc de produire des radicaux hydroxyles tant qu'il y a de l'eau oxygénée. Elle permet donc de produire en continu des radicaux hydroxyles.

La présente invention concerne également un procédé d'hydroxylation, d'alcoxylation ou d'oxycarbonylation d'une surface de polymères, lesdits polymères étant différents des polymères choisis parmi : le polyméthacrylate de méthyle (PMMA) et les polymères fluorocarbonés dans le cadre du procédé d'hydroxylation, ou de mélanges de polymères, lesdits polymères étant constitués de motifs monomériques dont au moins 50% parmi ceux-ci sont des motifs aliphatiques, pour obtenir une surface hydroxylée, alcoxylée ou oxycarbonylée,
ledit procédé étant caractérisé en ce qu'il consiste à faire réagir ladite surface avec des radicaux RO•, R représentant un atome d'hydrogène; un groupe alkyle comprenant de 2 à 15 atomes de carbone, un groupe acyle -COR' dans lequel R' représente un groupe alkyle comprenant de 2 à 15 atomes de carbone, et notamment un groupe butyle ou lauryle, ou un groupe aroyle -COAr dans lequel Ar représente un groupe aromatique comprenant de 6 à 15 atomes de carbone, et notamment un groupe phényle, lesdits radicaux RO• étant générés par voie électrochimique ou photochimique.

L'expression "surface hydroxylée" désigne une surface comportant des groupes hydroxyles (-OH).

L'expression "surface alcoxylée" désigne une surface comportant des groupes hydroxyles (-OR), R étant un groupe alkyle tel que défini ci-dessus.

L'expression "surface oxycarbonylée" désigne une surface comportant des groupes oxycarbonyles (-OCOR' ou -OCOAr, R' et Ar étant tels que définis ci-dessus). Selon un mode de réalisation particulier, les surfaces oxycarbonylées obtenues selon le procédé de l'invention comportent des groupes -COR' ou -COAr, R' et Ar étant tels que définis ci-dessus.

Les radicaux RO• sont obtenus par coupure du peroxyde RO-OR catalysée par le Fer (II).

Les radicaux HO•sont formés par coupure de l'eau oxygénée H₂O₂ catalysée par le Fer (II).

Un procédé d'hydroxylation préféré selon l'invention est caractérisé en ce qu'il consiste à faire réagir la surface avec des radicaux hydroxyles HO•.

La présente invention concerne également un procédé d'hydroxylation d'une surface de polymères, notamment hydrophobes, lesdits polymères étant constitués de motifs monomériques dont au moins 50% parmi ceux-ci sont des motifs de type alcane, pour obtenir une surface hydroxylée, ledit procédé étant caractérisé en ce qu'il consiste à faire réagir ladite surface avec des radicaux hydroxyles HO• obtenus par la mise en oeuvre de la réaction de Fenton, par voie électrochimique ou photochimique (réaction d'ElectroFenton ou réaction de PhotoFenton).

La présente invention concerne le procédé tel que défini ci-dessus, caractérisé en ce que les radicaux hydroxyles HO• sont obtenus par la mise en présence d'eau oxygénée et d'ions ferriques (Fe³⁺) ou ferreux (Fe²⁺).

Selon un mode de réalisation avantageux, le procédé de l'invention est caractérisé en ce que les radicaux hydroxyles HO• sont obtenus par la mise en oeuvre de la réaction d'ElectroFenton.

Selon un mode de réalisation avantageux, le procédé de l'invention, comprenant la mise en oeuvre de la réaction d'ElectroFenton, est caractérisé en ce que l'eau oxygénée est obtenue directement par la réduction électrochimique d'oxygène en milieu acide, et en ce que les radicaux hydroxyles HO• proviennent de la réaction des ions ferreux avec l'eau oxygénée.

L'expression "réduction électrochimique d'oxygène" désigne le transfert de deux électrons et de deux protons au dioxygène pour produire l'eau oxygénée.

L'expression "milieu acide" désigne un milieu dont le pH est inférieur à 7 et plus particulièrement est compris de 2 à 4, et de manière plus précise égal à 3.

La présente invention concerne également un procédé tel que défini ci-dessus, caractérisé en ce que les radicaux hydroxyles HO• sont obtenus par la mise en oeuvre de la réaction de PhotoFenton.

Selon un mode de réalisation avantageux, dans le cadre de la mise en oeuvre de la réaction de PhotoFenton, le procédé de l'invention est caractérisé en ce que l'eau oxygénée est ajoutée à la surface des polymères, et en ce que les radicaux hydroxyles HO• sont obtenus par la mise en présence de ladite surface avec une solution aqueuse comprenant l'eau oxygénée et un sel ferreux, notamment du chlorure ou du sulfate ferreux, et par l'irradiation de ladite surface et de ladite solution.

L'expression "irradiation de ladite surface et de ladite solution" désigne le fait d'éclairer la solution et la ou les surfaces avec une lampe émettant dans les longueurs d'onde convenables, particulièrement dans l'ultraviolet, c'est-à-dire à des longueur d'ondes inférieures à 400 nm.

Selon un autre mode de réalisation avantageux, la présente invention concerne le procédé tel que défini ci-dessus, caractérisé en ce que les polymères sont immergés dans une solution aqueuse comprenant l'eau oxygénée et un sel ferreux, notamment du chlorure ou du sulfate ferreux, et en ce que ladite solution et les polymères sont irradiés par lampe UV.

La présente invention concerne également un procédé tel que défini ci-dessus, caractérisé en ce que les polymères comprennent des motifs monomériques présentant une masse moléculaire variant d'environ 500 à environ 5 millions de daltons.

Une masse moléculaire de 500 daltons correspond à la limite basse des masses moléculaires des polymères et une masse moléculaire de 5 millions de daltons correspond à l'ordre de grandeur du polyéthylène UHMW (UltraHigh Molecular Weight) utilisé.

La présente invention concerne également un procédé tel que défini ci-dessus, caractérisé en ce que les polymères sont choisis parmi : le polyéthylène, le polypropylène, le polyisobutylène (P-IB), le polyméthylpentène, le polychlorure de vinyle (PVC), le polyacétate de vinyle (PVAC), le polybutyral de vinyle (PVB), le polyformal de vinyle (PVFM), le polyalcool vinylique (PVAL), les différents polyamides, les polyoxométhylènes (POM), les dérivés cellulosiques et le polyacrylonitrile (PAN).

Les polymères préférés pour la mise en oeuvre du procédé de l'invention sont choisis parmi les suivants :

| Polymère | Motif |
|---|---|
| Polyéthylène | -(CH₂)ₙ- |
| Polypropylène | 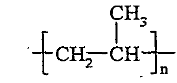 |
| Polyisobutylène | 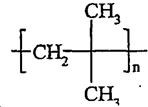 |
| Polyméthylpentène | 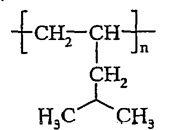 |
| Chlorure de polyvinyle | 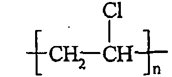 |
| Polyacétate de vinyle | 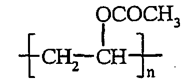 |
| Polyacrylonitrile | 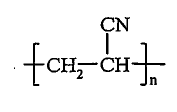 |
| Alcool polyvinylique | 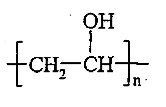 |
| Polybutyral de vinyle | 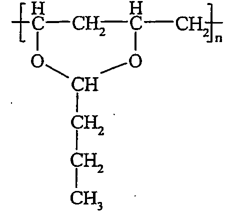 |
| Polyformal de vinyle | 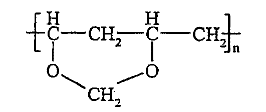 |
| Polyoxyméthylène | 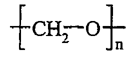 |
| Polyamide | 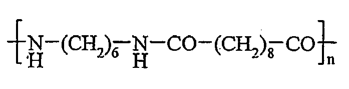 |

De préférence, les polymères utilisés pour la mise en oeuvre du procédé de l'invention sont des polyoléfines qui sont les polymères obtenus par polymérisations d'oléfines, c'est-à-dire de composés comportant au moins une double liaison dont l'ouverture conduit à la polymérisation.

De préférence, les polymères impliqués dans l'invention sont différents des polymères de polysiloxanes.

La présente invention concerne également un procédé tel que défini ci-dessus, caractérisé en ce que les polymères sont constitués de motifs monomériques comprenant au moins un motif aromatique, notamment un groupe aryle pendant, et au moins un motif de type alcane, lesdits polymères étant choisis parmi : les copolymères statistiques, les copolymères alternés et les copolymères à blocs (diblocs, triblocs, multiblocs ou radiaux).

Les copolymères alternés sont des polymères de la forme : -ABABABAB-.

Les copolymères blocs ou séquencés sont des polymères de la forme :
-AAAAAABBBBBBAAAAAABBBBBBB- ou
-AAAAAABBBBBBCCCCCCAAAAAABBBBBB-

Les copolymères statistiques sont des polymères de la forme :
-AABABBAAABABB-

Les copolymères biséquencés sont des polymères de la forme : -(A)ₙ-(B)ₜ-

Les copolymères triséquencés sont des polymères de la forme : -(A)ₘ-(B)ₙ-(A)ₜ-

Les copolymères en étoile (ou radiaux) sont des polymères de la forme : 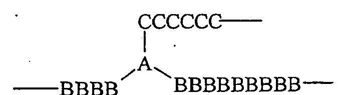 A, B et C représentant des motifs monomériques tels que définis ci-dessus.

La présente invention concerne également un procédé tel que défini ci-dessus, caractérisé en ce que la surface de polymères est sous la forme d'une feuille, d'un tricot, d'un tube, par exemple un cathéter, de fils, de clous ou vis, de billes, d'objets de formes diverses pouvant servir, par exemple, de prothèses ou de lentilles extra ou intraoculaires.

Selon un mode de réalisation préféré, le procédé de l'invention est caractérisé en ce qu'il ne comprend pas d'étape de réticulation.

La présente invention concerne également un procédé tel que défini ci-dessus, caractérisé en ce que l'étape consistant à faire réagir la surface avec les radicaux hydroxyles HO• est effectuée pendant environ 5 minutes à environ 5 heures.

La présente invention concerne également un procédé tel que défini ci-dessus, caractérisé en ce qu'il comprend une étape ultérieure de fonctionnalisation sur les groupes hydroxyles fixés sur la surface hydroxylée.

Parmi les réactions ultérieures de fonctionnalisation, on peut citer : la formation d'esters par réaction avec un acide carboxylique, d'éthers par réaction de Williamson avec un autre alcool, d'halogénures par réaction avec des acides halogénés ou PCl₅, de N-alkylamides par la réaction de Mitsunobu ou de sulfures par réaction de thiols.

De manière générale, la post-fonctionnalisation permet à l'homme de l'art de placer sur la surface la fonction organique souhaitée pour l'application visée, par exemple dans le domaine biomédical, l'adhésion ou la non-adhésion des protéines, l'accrochage de médicaments, d'antimicrobiens...

Les réactions de fonctionnalisation ultérieures permettent de conférer des propriétés spécifiques à la surface : par exemple en accrochant des molécules à activité biologique (par exemple des enzymes) ou à propriétés pharmaceutiques. Ceci peut être réalisé en liant le groupe OH sur la surface soit directement à la molécule souhaitée soit par l'intermédiaire d'un bras intermédiaire. On peut lier les fonctions alcools par formation d'ester (par réaction avec un anhydride, un chlorure d'acide ou même d'un acide), d'amides (par réaction avec des isocyanates) ou d'éthers (par réaction avec des halogénures d'alkyle).

La présente invention concerne également un procédé d'hydroxylation tel que défini ci-dessus, caractérisé en ce que l'angle de contact mesuré entre une goutte d'eau et la surface hydroxylée obtenue diminue de plus de 5°, notamment de plus de 10° par rapport à l'angle de contact mesuré entre une goutte d'eau et la surface non hydroxylée.

L'angle de contact est mesuré en déposant une goutte d'eau sur la surface du polymère avec une seringue, puis en mesurant à l'aide d'un microscope l'angle formé entre la surface du polymère et la tangente à la goutte à son point de contact avec le polymère.

Plus la surface est hydrophile, plus l'angle de contact mesuré est petit, et plus la surface est hydrophobe, plus l'angle de contact mesuré est grand.

La présente invention concerne également un procédé tel que défini ci-dessus, caractérisé en ce que les surfaces hydroxylées, alcoxylées ou oxycarbonylées obtenues sont stables dans le temps, notamment pendant plusieurs semaines, selon le test suivant :
Les spectres infrarouge des échantillons de PET et de PEEK, modifiés par réaction d'électroFenton soit pendant 10 minutes, soit pendant 120 minutes, puis trifluoroacétylés, sont réenregistrés après 74 jours. En enregistrant la différence des spectres (à t=0 et t=74 jours), on ne note pas de différence significative et en particulier pas de disparition des bandes caractéristiques des groupes trifluoroacétyles.

La présente invention concerne également des surfaces hydroxylées, alcoxylées ou oxycarbonylées obtenues par la mise en oeuvre du procédé de l'invention tel que défini ci-dessus.

Comme le montre la mesure des angles de contacts rapportés dans la partie expérimentale ci-après, les surfaces traitées selon le procédé d'hydroxylation de la présente invention sont bien devenues hydrophiles et seront par conséquent beaucoup plus biocompatibles.

La liaison entre le polymère et le groupe OH est une liaison covalente dont l'énergie est de l'ordre de 390 (CH₃OH) à 470 kJ/mol (C₆H₅OH).

### I - RÉACTION D'ELECTRO-FENTON

### Hydroxylation du Polypropylène (PP) du Polyéthylène (PE) et de l'Acrylonitrile-Butadiène-Styrène (ABS)

Substrats
- PP tricot et feuille Goodfellow PP 301400
- PE très haut poids moléculaire (feuille)(Goodfellow, ET301400)
- ABS plaque (Goodfellow, AB3030090) [obtenue en dispersant une phase élastomérique greffée (butadiène) dans une phase styrénique (SAN) obtenue par copolymérisation d'acrylonitrile avec le styrène]

### Dispositif électrochimique :

Cellule à compartiments non séparés.
Anode : carbone.
Cathode : feutre de carbone (environ 10 cm²).

La feuille ou le tricot de polymère sont serrés contre une feuille ou placés entre deux feuilles de feutre de carbone servant de cathode.
Solvant H₂SO₄ 0,1M amené à pH 3 avec de la soude
Catalyseur Fe²⁺ 0,5 mM (FeSO₄, 7H₂O)
Bullage continu d'air
*Méthode Galvanostatique :* Courant constant : 10 mA ou 5 mA selon les expériences
*Méthode Potentiostatique :* Potentiel constant E = -0,6V/SCE

### A) Hydroxylation du PP

### Hydroxylation d'une feuille de PP (Goodfellow)

En galvanostatique i = 5 mA, 2 heures, la contre électrode est court-circuitée sur la référence. Le potentiel de la cathode augmente d'environ -0,6V/SCE au début de l'expérience à environ -2V/SCE en fm d'électrolyse, à ce potentiel on doit réduire les protons et donc réduire l'efficacité de la réduction de l'oxygène. Les échantillons sont soigneusement rincés à l'eau distillée 10 minutes sous ultrasons, puis 10 minutes dans l'acétonitrile (pour analyse) et séchés à 40°C sous vide pendant une nuit.

En potentiostatique, le courant diminue d'environ 30 à environ 10 mA. Les échantillons sont traités comme ci-dessus.

Pour l'analyse IR, les échantillons sont traités une nuit dans une solution d'anhydride trifluoroacétique (1 mL) dans l'éther (30 mL), rincés à l'acétonitrile puis séchés sous vide une nuit. Les résultats sont rassemblés dans le Tableau 1.

**Tableau 1. Analyse IR d'une feuille de PP hydroxylée puis trifluoroacétylée***

| Position des bandes en cm⁻¹ | Attribution | Par comparaison |
|---|---|---|
| 1794 | C=O | ≈ 1813 cm⁻¹ pour (CF₃CO)₂O |
| | | ≈ 1780 cm⁻¹ pour CF₃COOH |
| 1206 | CF3 | ≈ 1160, 1240 cm⁻¹ pour (CF₃CO)₂O |
| 1179 | | ≈ 1190, 1240 cm⁻¹ pour CF₃COOH |

| | | |
|---|---|---|
| Référence : feuille de PP non traitée. | | |

La bande CF₃ est plus grande sur le spectre correspondant à l'électrolyse potentiostatique que sur celle en galvanostatique, il semble que le rendement soit plus élevé en potentiostatique, par contre il n'y a aucune différence en ce qui concerne la position des bandes.

L'analyse ToF-SIMS confirme bien la trifluorométhylation de la surface

-OH + CF3(C=O)O(O=C)CF₃→ -O(C=O)CF₃

(voir fragments décrits dans le Tableau 2 ci-après).

**Tableau 2 Analyse ToF-SIMS d'une feuille de PP hydroxylée puis trifluoroacétylée**

| m/z | Attribution^{a)} |
|---|---|
| 19 | F⁻ |
| 97 | C(=O)CF₃⁻ ou CH₂CH₂CF₃^{-b}) |
| 113 | OC(=O)CF₃⁻ |
| 69 | CF₃⁺ |
| 95 | CH=CHCF₃⁺ |
| 97 | CH₂CH₂CF₃⁺ ou C(=O)CF₃^{-b}) |
| 109 | CH₂CH=CHCF₃⁺ |
| 111 | CH₂CH₂CH₂CF₃^{+b}) |
| 123 | CH₂CH₂CH=CHCF₃^{+b)} |
| 125 | CH₂CH₂CH₂CH₂CF₃^{+b)} |
| 147 | C≡CCH₂CH₂CH=CHCF₃^{+b}) |
| 207 | CH₂(CH₂)₅CH=CHCF₃^{+b)}, |
| 221 | CH₂(CH₂)₆CH=CHCF₃^{+b}) |

| | |
|---|---|
| a) ou isomère, b) CH₂CH₂ et C=O ayant même masse, il serait possible d'écrire les ions m/z = 97 (positifs et négatifs) sous les deux formes isomère indiquées. L'ion négatif est attribué au groupe trifluoroacétyle. Pour l'ion positif, la présence de l'ion à m/z =95 semble indiquer la perte de deux hydrogènes et permet donc d'attribuer le pic à l'ion trifluoroéthyle. | |

La présence des groupes trifluorométhyles (et particulièrement de chaînes alkyles trifluorométhylés) confirme que la surface a bien été hydroxylée puis trifluorométhylée. Cependant, il y a probablement au cours de l'analyse un réarrangement des ions avec perte de CO₂ puisqu'on n'observe pas de fragments de type (CH₂)ₙOC(=O)CF₃.

L'analyse de l'angle de contact de l'eau mesuré du PP non traité est de 124°, après traitement il diminue à 102° immédiatement après dépôt de la goutte, et continue à décroître lentement par la suite : 73° après 10 et 20 minutes (sans évaporation notable de la goutte).

### Hydroxylation du tricot

Electrolyse 1 heure. Méthode galvanostatique : i = 10mA
L'analyse IR de la surface présente par différence avec un échantillon non traité une bande à 3419 cm⁻¹ qui peut correspondre à une vibration d'élongation OH, une bande faible à 1037cm⁻¹ peut être attribuée à une déformation C-OH.

Par ToF-SIMS on observe des pics O et OH nettement plus grands que sur la référence ainsi que les pics non présents sur la référence rassemblés dans le Tableau 3.

**Tableau 3. Analyse du Tricot Sofradim hydroxylé par ToF-SIMS**

| m/z | Attribution |
|---|---|
| 32 | CH₃O⁻ |
| 60 | HO-HC=CH-OH- |
| 79 | C₅H₃OH⁻ |
| 113 | C₇H₁₃OH⁻ |

Après trifluorométhylation, on observe les pics fluorés : F⁻, CF₃⁻, OCOCF₃⁻, C₇H₁₅OCOCF₃⁻. Ce dernier pic est particulièrement intéressant, il contient une partie du polymère et la fonction OH qui a été trifluoroacétylée, montrant bien ainsi que la fonction OH est bien attachée de manière covalente à la surface. On note quelques différences entre les spectres de la feuille et du tricot, cela peut être dû soit à la reproductibilité des expériences, soit à une réactivité différente des deux substrats bien qu'ils soient de composition chimique identique.

Sur un tricot, l'angle de contact est très difficile à mesurer, cependant, après hydroxylation, la goutte d'eau traverse la surface.

### B) Hydroxylation du PE

La modification de la surface a été réalisée dans une solution d'acide sulfurique 0,1N amenée à pH 3 par addition de soude, en présence de FeSO₄ 0,5mM, avec bullage d'air, en régime potentiostatique à -0,6 V/SCE pendant 2 heures.

L'angle de contact avec l'eau a été mesuré avant et après greffage : il passe d'environ 91° à environ 70°. Ceci montre bien que la surface du polypropylène a été hydroxylée.

Le spectre IR de la surface hydroxylée a été enregistré.

**Tableau 4. Spectre IR de PE après traitement***

| Position des bandes en cm⁻¹ | Attribution |
|---|---|
| 3310 | O-H |
| 1047 | C-O alcool primaire |

| | |
|---|---|
| * après soustraction d'une référence non traitée, échantillons séchés sous vide à 40°C pendant deux jours, pour s'assurer que les bandes OH ne proviennent pas de l'humidité superficielle. | |

L'échantillon est trifluoroacétylé comme ci-dessus. Pour obtenir une référence, on fait subir le traitement de trifluoroacétylation à un échantillon n'ayant pas été hydroxylé.

**Tableau 5. Analyse IR d'une feuille de PE hydroxylée puis trifluoroacétylée***

| Position des bandes en cm⁻¹ | Attribution | Par comparaison |
|---|---|---|
| 1742 | C=O | ≈1813 cm⁻¹ pour (CF₃CO)₂O |
| | | ≈1780 cm⁻¹ pour CF₃COOH |
| 1240 | CF3 | ≈1160, 1240 cm⁻¹ pour (CF₃CO)₂O |
| 1170 | | ≈1190, 1240 cm⁻¹ pour CF₃COOH |

| | | |
|---|---|---|
| Référence : feuille de PE non hydroxylée et ayant subi le traitement de trifluoroacétylation. | | |

**Tableau 6. Analyse ToF-SIMS d'une feuille de PE hydroxylée puis trifluoroacétylée**

| m/z | Attribution |
|---|---|
| 19 | F- |
| 69 | CF₃⁻; CF₃⁺ |
| 97 | C(=O)CF₃⁻; C(=O)CF₃⁺ |
| 113 | OC(=O)CF₃⁻ |
| 119 | (CH₂)₂CF₂H⁻ |
| 131 | C₃H₄OC(=O)CF₃⁺ |
| 155 | (CH₂)₃OC(=O)CF₃⁺ |
| 169 | (CH₂)₄OC(=O)CF₃⁺ |
| 181 | C₅H₈OC(=O)CF₃⁺ |
| 193 | C₆H₈OC(=O)CF₃⁺ |
| 265 | CH₃(CH₂)₁₀OC(=O)CF₃⁻ |

La variation de l'angle de contact, les spectres IR et tout particulièrement les spectres ToF-SIMS présentant des fragments AlkylOC(=O)CF₃, attestent bien de l'hydroxylation et de la post-fonctionnalisation de la surface.

### C) Hydroxylation de ABS

Dans les même conditions que PE.
L'angle de contact avec l'eau a été mesuré avant et après greffage : il passe d'environ 69° à environ 37°.
Le spectre IR de la surface hydroxylée a été enregistré.

**Tableau 7. Spectre IR de ABS après traitement.**

| Position des bandes en cm⁻¹ | Attribution |
|---|---|
| 3240 | O-H |
| 1050 | C-O alcool primaire |

| | |
|---|---|
| * après soustraction d'une référence non traitée, échantillons séchés sous vide à 40°C pendant deux jours pour s'assurer que les bandes OH ne proviennent pas de l'humidité superficielle. | |

L'échantillon est trifluoroacétylé comme ci-dessus. Pour obtenir une référence, on fait subir le traitement de trifluoroacétylation à un échantillon n'ayant pas été hydroxylé.

**Tableau 8. Analyse IR d'une feuille de ABS hydroxylée puis trifluoroacétylée***

| Position des bandes en cm⁻¹ | Attribution | Par comparaison |
|---|---|---|
| 1765 | C=O | ≈1813 cm⁻¹ pour (CF₃CO)₂O |
| | | ≈ 1780 cm⁻¹ pour CF₃COOH |
| 1245 ep | CF3 | ≈ 1160, 1240 cm⁻¹ pour (CF₃CO)₂O |
| 1160 ep | | ≈ 1190, 1240 cm⁻¹ pour CF₃COOH |

| | | |
|---|---|---|
| Référence : feuille de ABS non hydroxylée et ayant subit le traitement de trifluoroacétylation. | | |

**Tableau 9. Analyse ToF-SIMS d'une feuille de ABS hydroxylée puis trifluoroacétylée***

| m/z | Attribution |
|---|---|
| 19 | F⁻ |
| 69 | CF3⁻ |
| 97 | C(=O)CF3⁻ |
| 145 | OC(=O)CF3- |
| 228 | NC-(CH₂)₅-OC(=O)CF₃⁺ |

### Stabilité du greffage dans le temps

Apèrs 74 jours, les spectres infrarouge d'un échantillon de PP modifié par réaction d'électroFenton soit pendant 10 minutes, soit pendant 120 minutes puis trifluoroacétylé ont été réenregistrés. En enregistrant la différence des spectres (à t=0 et t=74 jours), on ne note pas de différence significative et en particulier pas de disparition des bandes caractéristiques des groupes trifluoroacétyles.

### II - RÉACTION DE PHOTO-FENTON

### Hydroxylation du Polypropylène (PP) du Polyéthylène (PE) et de l'Acrylonitrile-Butadiène-Styrène (ABS)

### A) Hydroxylation du Polypropylène (PP)

Un réacteur en verre de 2L muni d'une pompe de circulation, d'une double enveloppe de thermostatation et d'une lampe mercure basse pression placée au centre du réacteur dans un tube en quartz est rempli de 2 L de solution HCl 1 mM dans l'eau, de 1 g de chlorure ferrique et de 2 mL d'eau oxygénée. Les échantillons de polymères sont suspendus dans la solution. On met en marche la pompe et l'irradiation ; après 2h 30, on arrête l'irradiation. Les échantillons sont rincés 15 minutes dans l'eau distillée sous ultrasons, puis à l'acétone et séchés sous vide à 40°C pendant une nuit.

L'analyse des échantillons par IR permet de voir des bandes attribuables à des groupes OH à 3343 et 3230 cm⁻¹ (après soustraction su spectre du PP lui-même).

La mesure des angles de contact de l'eau a été effectuée avant et après traitement.

**Tableau 10. Angles de contact d'échantillons de polymères traités par PhotoFenton.**

| Echantillon | Angle de contact avant traitement | Angle de contact après traitement |
|---|---|---|
| PP | 124 | 124^{a)} 86 (après 10')^{b)} 80 (après 20') |

| | | |
|---|---|---|
| a) immédiatement après dépôt de la goutte b) sans diminution notable de la taille de la goutte. | | |

Les échantillons sont ensuite traités à l'anhydride trifluoroacétique (0,4 mL dans 10 mL d'éther) et analysés par IR. On observe bien les bandes vers 1206-1254 cm⁻¹ et 1165-1185 cm⁻¹ qui sont attribuées aux vibrations du groupe CF₃ par comparaison avec les spectres de l'acide et de l'anhydride trifluoroacétique. La vibration correspondant au groupement carbonyle (C=O)CF₃ est observable. Ces spectres confirment bien la modification de la surface des polymères.

**Tableau 11. Spectres IR des échantillons trifluoroacétylés.**

| Echantillon | Absorption IR en cm⁻¹ | Attribution |
|---|---|---|
| PP^{a} | ≈1800 vw 1206 s 1166m | C=O (≈ 1790 cm⁻¹ pour CF₃COOH) CF₃ (1248 pour CF3CO₂O et1240 pour CF₃COOH) CF₃ (1195 pour CF3CO)₂O et 1177 pour CF₃COOH) |

**Tableau 12. Spectres ToF-SIMS des échantillons trifluoroacétylés.**

| Echantillon | m/z | Attribution |
|---|---|---|
| PP^{a} | 19 | F⁻ |
| | 69 | CF₃⁻,CF₃⁺ |
| | 113 | [O(C=O)CF₃]⁻ |
| | 182 | [CF₃(C=O)O CHCH₃CH₂CCH₃]⁻ |

Les spectres ToF-SIMS confirment bien le greffage des polymères par des groupes OCF₃ après traitement à l'anhydride acétique, donc l'hydroxylation des surfaces.

### B) Hydroxylation du Polyéthylène (PE)

L'hydroxylation et la trifluoroacétylation ont été effectuées dans les mêmes conditions que le polypropylène. L'échantillon est analysé par ToF-SIMS

**Tableau 13. Spectres ToF-SIMS des échantillons trifluoroacétylés.**

| Echantillon | m/z | Attribution |
|---|---|---|
| PE^{a} | 19 | F⁻ |
| | 69 | CF₃⁻, CF₃⁺ |
| | 97 | C(=O)CF₃⁻ |
| | 113 | [O(C=O)CF₃]⁻ |
| | 182 | [CF₃(C=O)O CH(CH₂)₄]⁻ |

Le Tableau 13 indique bien le greffage du polyéthylène par des groupes OH et les trifluoroacétylations ultérieures. En particulier, le fragment à m/z = 1182 présente un fragment de chaîne de PE portant un groupe trifluoroacétyle.

### C) Hydroxylation du ABS

Dans les même conditions que le PP, les échantillons sont analysés par ToF-SIMS.

**Tableau 14. Spectres ToF-SIMS des échantillons trifluoroacétylés.**

| Echantillon | m/z | Attribution |
|---|---|---|
| PE^{a} | 19 | F⁻ |
| | 69 | CF₃⁻, CF₃⁺ |
| | 97 | C(=O)CF₃⁻ |
| | 113 | [O(C=O)CF₃]⁻ |
| | 207 | OC(=O)CF₃ |

Le spectre confirme bien l'hydroxylation et la trifluoroacétylation de l'ABS.

### Analogue de la réaction de Fenton avec le peroxyde de Benzoyle

Dans cette réaction, on remplace l'eau oxygénée par le peroxyde de benzoyle. Deux échantillons de PP et de PE sont enveloppés dans du feutre de carbone, ces emballages sont utilisés comme cathode. Une solution est préparée en introduisant 400 mg de peroxyde de benzoyle (solution saturée) et 55 mg de Fe₂SO₄, 7H₂O dans 400 ml d'une solution H₂SO₄ 0,1N amenée à pH 3 par addition de soude. La solution est désoxygénée à l'argon. Le potentiel est fixé à E = -0,6 V/SCE pendant deux heures. Les échantillons sont ensuite rincés à l'eau du robinet, deux fois à l'eau distillée sous ultrasons (10 minutes) et une fois à l'acétone sous ultrasons et séchés sous vide.
Les échantillons sont examinés par ToF-SIMS.

**Tableau 15. Spectres Tof-SIMS d'échantillons traités par le peroxyde de benzoyle dans les conditions de la réaction de Fenton**

| m/z | Attribution |
|---|---|
| PE | |
| 105 | C₆H₅C(=O)⁺ |
| 120 | C₆H₅C(=O)CH₃⁺ |
| 163 | C₆H₅C(=O)(CH2)₃⁺ |
| PP | C₆H₅C(=O)⁺ |
| 105 | |
| 147 | CH₂CH(CH₃)C(=O)C₆H₅⁻ |
| 207 | CH₃CH(CH₃)CH₂CH(CH₃)OC(=O)C₆H₅ |

Le Tableau 15 indique donc bien le greffage par des groupes C₆H₅C(=O) ou C₆H₅C(=O)O pour le PE et le PP.

## Revendications

1. Utilisation de radicaux RO•, R représentant un atome d'hydrogène, un groupe alkyle comprenant de 2 à 15 atomes de carbone, un groupe acyle -COR' dans lequel R' représente un groupe alkyle comprenant de 2 à 15 atomes de carbone, ou un groupe aroyle -COAr dans lequel Ar représente un, groupe aromatique comprenant de 6 à 15 atomes de carbone, pour l'hydroxylation, l'alcoxylation ou l'oxycarbonylation de surfaces de polymères, lesdits polymères étant différents des polymères choisis parmi :
le polyméthacrylate de méthyle (PMMA) et les polymères fluorocarbonés lorsque R représente un atome d'hydrogène, ou de mélanges de polymères, notamment hydrophobes, lesdits polymères étant constitués de motifs monomériques dont au moins 50% parmi ceux-ci sont des motifs aliphatiques et lesdits radicaux RO• étant générés par mise en oeuvre d'une réaction de Fenton par voie électrochimique ou photochimique.

2. Utilisation selon la revendication 1 pour l'hydroxylation, l'alcoxylation ou l'oxycarbonylation de surfaces de polymères, la réaction d'hydroxylation étant effectuée sur des polymères différents des polymères choisis parmi : le polyméthacrylate de méthyle (PMMA) et les polymères fluorocarbonés, ou de mélanges de polymères, lesdits polymères étant constitués de motifs monomériques dont au moins 50% parmi ceux-ci sont des motifs aliphatiques, **caractérisée en ce que** la réaction de Fenton est mise en oeuvre par voie électrochimique ou par voie photochimique.

3. Procédé d'hydroxylation, d'alcoxylation ou d'oxycarbonylation d'une surface de polymères, lesdits polymères étant différents des polymères choisis parmi : le polyméthacrylate de méthyle (PMMA) et les polymères fluorocarbonés dans le cadre du procédé d'hydroxylation, ou de mélanges de polymères, lesdits polymères étant constitués de motifs monomériques dont au moins 50% parmi ceux-ci sont des motifs aliphatiques, pour obtenir une surface hydroxylée, alcoxylée ou oxycarbonylée,
ledit procédé étant **caractérisé en ce qu'**il consiste à faire réagir ladite surface avec des radicaux RO•, R représentant un atome d'hydrogène, un groupe alkyle comprenant de 2 à 15 atomes de carbone, un groupe acyle -COR' dans lequel R' représente un groupe alkyle comprenant de 2 à 15 atomes de carbone, et notamment un groupe butyle ou lauryle, ou un groupe aroyle -COAr dans lequel Ar représente un groupe aromatique comprenant de 6 à 15 atomes de carbone, et notamment un groupe phényle, et lesdits radicaux RO• étant générés par mise en oeuvre d'une réaction de Fenton par voie électrochimique ou photochimique.

4. Procédé d'hydroxylation selon la revendication 3, **caractérisé en ce qu'**il consiste à faire réagir la surface avec des radicaux HO•.

5. Procédé d'hydroxylation d'une surface de polymères selon la revendication 3 **caractérisé en ce que** lesdits polymères étant constitués de motifs monomériques dont au moins 50% parmi ceux-ci sont des motifs aliphatiques et étant différents des polymères choisis parmi : le polyméthacrylate de méthyle (PMMA) et les polymères fluorocarbonés, pour obtenir une surface hydroxylée, lesdits polymères étant notamment choisis parmi : le polyéthylène, le polypropylène, le polyisobutylène (P-IB), le polyméthylpentène, le polychlorure de vinyle (PVC), le polyacétate de vinyle (PVAC), le polybutyral de vinyle (PVB), le polyformal de vinyle (PVFM), le polyalcool vinylique (PVAL), les différents polyamides, les polyoxométhylènes (POM), les dérivés cellulosiques et le polyacrylonitrile (PAN),
ledit procédé étant **caractérisé en ce qu'**il consiste à faire réagir ladite surface avec des radicaux hydroxyles HO• obtenus par la mise en oeuvre de la réaction de Fenton, par voie électrochimique ou photochimique (réaction d'ElectroFenton ou réaction de PhotoFenton).

6. Procédé selon la revendication 5, **caractérisé en ce que** les radicaux hydroxyles HO• sont obtenus par la mise en présence d'eau oxygénée et d'ions ferriques (Fe³⁺) ou ferreux (Fe²⁺).

7. Procédé selon l'une quelconque des revendications 3 à 6, **caractérisé en ce que** les polymères comprennent des motifs monomériques présentant une masse moléculaire variant de 500 à 5 millions de daltons.

8. Procédé selon l'une quelconque des revendications 3 à 7, **caractérisé en ce que** la surface de polymères est sous la forme d'une feuille, d'un tricot, d'un tube, par exemple un cathéter, de fils, de clous ou vis, de billes, d'objets de formes diverses pouvant servir de prothèses ou de lentilles extra ou intraoculaires.

9. Procédé selon l'une quelconque des revendications 5 à 8, **caractérisé en ce que** l'étape consistant à faire réagir la surface avec les radicaux hydroxyles HO• est effectuée pendant 5 minutes à 5 heures.

10. Procédé selon l'une quelconque des revendications 5 à 9, **caractérisé en ce qu'**il comprend une étape ultérieure de fonctionnalisation sur les groupes hydroxyles fixés sur la surface hydroxylée.

## Claims

1. Use of RO• radicals, wherein R represents a hydrogen atom, an alkyl group comprisinging 2 to 15 carbon atoms, an acyl group -COR' in which R' represents an alkyl group having 2 to 15 carbon atoms, or a aroyle group -COAr in which Ar represents an aromatic group having 6 to 15 carbon atoms, for hydroxylation, alkoxylation or oxycarbonylation of polymer surfaces, said polymers being different from polymers chosen among: polymethylmethacrylate (PMMA) and fluorocarbon polymers when R represents a hydrogen, or of polymer mixture surfaces, in particular hydrophobic ones, said polymers consisting in monomeric units of which at least 50% among these are aliphatic units, and said RO• radicals being generated by performing a Fenton reaction by electrochemical or photochemical means.

2. Use according to claim 1 for hydroxylation, alkoxylation or oxycarbonylation of polymer surfaces, the hydroxylation reaction being performed on polymers being different from polymers chosen among: polymethylmethacrylate (PMMA) and fluorocarbon polymers when R represents a hydrogen, or of polymer mixture surfaces, in particular hydrophobic ones, said polymers consisting in monomeric units of which at least 50% among these are aliphatic units, **characterized in that** Fenton reaction is performed by electrochemical or photochemical route.

3. A process for hydroxylation, alkoxylation or oxycarbonylation of polymer surfaces, the said polymers being different from polymers chosen among:
polymethylmethacrylate (PMMA) and fluorocarbon polymers for the hydroxylation process, or of polymer mixture surfaces, said polymers consisting in monomeric units of which at least 50% among these are aliphatic units, to obtain a hydroxylated, alkoxylated or oxycarbonylated surface,
said process being **characterised in that** it consists in reacting said surface with RO• radicals, R chosen among a hydrogen atom, an alkyl group having 2 to 15 carbon atoms, an acyl group -COR' in which R' represents an alkyl group comprising 2 to 15 carbon atoms, and in particular a butyl or lauryl group, or an aroyl group -COAr in which Ar represents an aromatic group having 6 to 15 carbon atoms, and in particular a phenyl group, and said RO• radicals being generated by carrying out Fenton reaction by electrochemical or photochemical route.

4. A hydroxylation process according to claim 3, **characterised in that** it consists of reacting the surface with HO• hydroxyl radicals.

5. A hydroxylation process of a polymer surface, according to claim 3 **characterized in that** said polymers consisting in monomeric units of which at least 50% among these are aliphatic units and being different from polymers chosen among:
polymethylmethacrylate (PMMA) and fluorocarbon polymers, to obtain a hydroxylated surface, the polymers being in particular chosen among: polyethylene, polypropylene, polyisobutylene (P-IB), polymethylpentene, polyvinyl chloride (PVC), polyvinyl acetate (PVAC), polyvinyl butyral (PVB), polyvinyl formal (PVFM), polyvinyl alcohol (PVAL), different polyamides, polyoxomethylenes (POM), cellulose derivatives et polyacrylonitrile (PAN),
said process being **characterised in that** it consists in reacting said surface with HO• hydroxyl radicals obtained by carrying out the Fenton reaction, by electrochemical or photochemical means (ElectroFenton or PhotoFenton reaction).

6. A process according to claim 5, **characterised in that** the HO• hydroxyl radicals are obtained by mixing hydrogen peroxide and ferric (Fe³⁺) or ferrous (Fe²⁺) ions.

7. A process according to any of claims 3 to 6, **characterised in that** the polymers contain monomeric units presenting a molecular weight from 500 to 5 million daltons.

8. A process according to any of claims 3 to 7, **characterised in that** the surface of the polymers is in a form of a sheet, a knitted material, a tube, for example a catheter, strands, nails or screws, balls, objects of different forms being able to serve as prostheses or extra or intraocular lenses.

9. A process according to any of claims 5 to 8, **characterised in that** the step of reacting the surface with the HO• hydroxyl radicals is carried out for 5 minutes to 5 hours.

10. A process according to any of claims 5 to 9, **characterised in that** it involves a subsequent functionalisation step on the hydroxyl groups linked to the hydroxylated surface.

## Patentansprüche

1. Verwendung von Radikalen RO•, wobei R für ein Wasserstoffatom, eine Alkylgruppe, die 2 bis 15 Kohlenstoffatome umfasst, eine Acylgruppe -COR', in der R' für eine Alkylgruppe steht, die 2 bis 15 Kohlenstoffatome umfasst, oder eine Aroylgruppe -COAr steht, in der Ar für eine aromatische Gruppe steht, die 6 bis 15 Kohlenstoffatome umfasst, zur Hydroxylierung, Alkoxylierung oder Oxycarbonylierung von Polymeroberflächen, wobei die Polymere verschieden sind von den Polymeren, die ausgewählt sind aus:
Polymethylmethacrylat (PMMA) und Fluorcarbonpolymeren, wenn R für ein Wasserstoffatom steht, oder Polymergemischen, insbesondere hydrophoben Polymergemischen, wobei die Polymere aus monomeren Struktureinheiten bestehen, von denen mindestens 50 % aliphatische Struktureinheiten sind und die Radikale RO• mittels Durchführung einer Fentonreaktion auf elektrochemischem oder photochemischem Weg erzeugt werden.

2. Verwendung nach Anspruch 1 zur Hydroxylierung, Alkoxylierung oder Oxycarbonylierung von Polymeroberflächen, wobei die Hydroxylierungsreaktion an Polymeren ausgeführt wird, die verschieden sind von den Polymeren, die ausgewählt sind aus:
Polymethylmethacrylat (PMMA) und Fluorcarbonpolymeren oder Polymergemischen, wobei die Polymere aus monomeren Struktureinheiten bestehen, von denen mindestens 50 % aliphatische Struktureinheiten sind, **dadurch gekennzeichnet, dass** die Fentonreaktion auf elektrochemischem Weg oder photochemischem Weg durchgeführt wird.

3. Verfahren zur Hydroxylierung, Alkoxylierung oder Oxycarbonylierung einer Polymeroberfläche, wobei die Polymere verschieden sind von den Polymeren, die ausgewählt sind aus: Polymethylmethacrylat (PMMA) und den Fluorcarbonpolymeren im Rahmen des Hydroxylierungsverfahrens, oder Polymergemischen, wobei die Polymere aus monomeren Struktureinheiten bestehen, von denen mindestens 50 % aliphatische Struktureinheiten sind, um eine hydroxylierte, alkoxylierte oder oxycarbonylierte Oberfläche zu erhalten, wobei das Verfahren **dadurch gekennzeichnet ist, dass** es darin besteht, die Oberfläche mit Radikalen RO• umzusetzen, wobei R für ein Wasserstoffatom, eine Alkylgruppe, die 2 bis 15 Kohlenstoffatome umfasst, eine Acylgruppe -COR', in der R' für eine Alkylgruppe steht, die 2 bis 15 Kohlenstoffatome umfasst, und insbesondere eine Butyl- oder Laurylgruppe oder eine Aroylgruppe -COAr, in der Ar für eine aromatische Gruppe steht, die 6 bis 15 Kohlenstoffatome umfasst, und insbesondere eine Phenylgruppe steht, und wobei die Radikale RO• mittels der Durchführung einer Fentonreaktion auf elektrochemischem oder photochemischem Weg erzeugt werden.

4. Hydroxylierungsverfahren nach Anspruch 3, **dadurch gekennzeichnet, dass** es darin besteht, die Oberfläche mit den Radikalen HO• umzusetzen.

5. Verfahren zur Hydroxylierung einer Polymeroberfläche nach Anspruch 3, **dadurch gekennzeichnet, dass** die Polymere aus monomeren Struktureinheiten bestehen, von denen 50 % aliphatische Struktureinheiten sind, und die verschieden sind von den Polymeren, die ausgewählt sind aus: Polymethylmethacrylat (PMMA) und Fluorcarbonpolymeren, um eine hydroxylierte Oberfläche zu erhalten, wobei die Polymere insbesondere ausgewählt sind aus: Polyethylen, Polypropylen, Polyisobutylen (P-IB), Polymethylpenten,
Polyvinylchlorid (PVC), Polyvinylacetat (PVAC), Polyvinylbutyral (PVB), Polyvinylformal (PVFM), Polyvinylalkohol (PVAL), verschiednen Polyamiden, Polyoxomethylenen (POM), Zellulosederivaten und Polyacrylnitril (PAN),
wobei das Verfahren **dadurch gekennzeichnet ist, dass** es darin besteht, die Oberfläche mit Hydroxylradikalen HO• umzusetzen, die mittels der Durchführung der Fentonreaktion auf elektrochemischem oder photochemischem Weg (ElectroFenton-Reaktion oder PhotoFenton-Reaktion) erhalten wurden.

6. Verfahren nach Anspruch 5, **dadurch gekennzeichnet, dass** die Hydroxylradikale HO• durch das Mischen von Wasserstoffperoxid und Eisen(III)-Ionen (Fe₃₊) oder Eisen(II)-Ionen (Fe₂₊) erhalten werden.

7. Verfahren nach einem der Ansprüche 3 bis 6, **dadurch gekennzeichnet, dass** die Polymere monomere Struktureinheiten umfassen, die ein Molekülgewicht aufweisen, das zwischen 500 und 5 Millionen Dalton variiert.

8. Verfahren nach einem der Ansprüche 3 bis 7, **dadurch gekennzeichnet, dass** die Polymeroberfläche die Form eines Blatts, eines Gestricks oder einer Röhre, zum Beispiel eines Katheters, von Fäden, Nägeln oder Schrauben, Kugeln, Gegenständen mit diversen Formen haben kann, die als Prothesen oder extra- oder intraokuläre Linsen dienen können.

9. Verfahren nach einem der Ansprüche 5 bis 8, **dadurch gekennzeichnet, dass** der Schritt der darin besteht, die Oberfläche mit den Hydroxylradikalen HO• umzusetzen, 5 Minuten bis 5 Stunden lang ausgeführt wird.

10. Verfahren nach einem der Ansprüche 5 bis 9, **dadurch gekennzeichnet, dass** es einen nachfolgenden Schritt der Funktionalisierung an den Hydroxylgruppen umfasst, die an der hydroxylierten Oberfläche fixiert sind.
